(19) Europäisches Patentamt
European Patent Office
Office européen des brevets

(11) **EP 1 983 341 A1**

(12) **EUROPÄISCHE PATENTANMELDUNG**

(43) Veröffentlichungstag:
**22.10.2008 Patentblatt 2008/43**

(51) Int Cl.:
**G01N 33/18** (2006.01)     **G01N 35/00** (2006.01)
**G01N 31/22** (2006.01)

(21) Anmeldenummer: **08103540.4**

(22) Anmeldetag: **15.04.2008**

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(30) Priorität: **19.04.2007 EP 07106535**

(71) Anmelder: **Hach Lange GmbH**
**40549 Düsseldorf (DE)**

(72) Erfinder:
• **Huenig, Isabel**
**01159, Dresden (DE)**
• **Farjam, Aria**
**40223, Düsseldorf (DE)**
• **Frömel, Rainer**
**53842, Troisdorf (DE)**

(74) Vertreter: **Fitzner, Uwe**
**Dres. Fitzner & Münch**
**Patent- und Rechtsanwälte**
**Hauser Ring 10**
**40878 Ratingen (DE)**

(54) **Verfahren zur Bestimmung der Konzentration von Analyten**

(57)     Die vorliegende Erfindung betrifft ein Verfahren zur Bestimmung der Konzentration von Analyten, wobei nacheinander
i) der Analyt mit einem Indikator in Kontakt gebracht wird,
ii) eine Bindung und/oder Anreicherung des Indikators, der mit dem Analyten reagiert hat, auf den magnetischen Partikeln stattfindet, sofern der Indikator nicht bereits auf den magnetischen Partikeln fixiert war,
iii) nach der Reaktion des Indikators mit dem Analyten und der Bindung und/oder Anreicherung des Indikators auf den magnetischen Partikeln, sofern der Indikator nicht bereits auf den magnetischen Partikeln fixiert war, mittels eines Magnetfeldes eine Aufkonzentrierung der Partikel aus der Lösung erfolgt und
iv) die Veränderung der magnetischen Partikel mittels optischer Methoden gemessen wird und wobei
- der Indikator
a) als Bestandteil eines Feststoffgemisches vorgelegt wird, das den Indikator und magnetische Partikel enthält und weitere Substanzen enthalten kann, oder
b) als Bestandteil eines Feststoffgemisches vorgelegt wird, das den Indikator und magnetische Partikel enthält und weitere Substanzen enthalten kann, wobei der Indikator auf die Oberfläche der magnetischen Partikel aufgebracht ist, und

- magnetische Partikel eingesetzt werden, welche einen Farbton aufweisen, welche nach dem CIELAB-System einen Wert von $L^* > 30$ aufweisen oder die weiß oder grau und/oder durchsichtig, bevorzugt weiß oder grau, sind.

Fig. 2

**Beschreibung**

Diese Anmeldung nimmt die Priorität der Europäischen Anmeldung mit der Anmeldenummer 07 106 535.3 in Anspruch.

Die vorliegende Erfindung betrifft ein Verfahren zur Bestimmung der Konzentration von Analyten, insbesondere in Wasser mittels optischer Messverfahren sowie magnetische Partikel zur Durchführung desselben.

Aus dem Stand der Technik sind verschiedene Verfahren zur Bestimmung der Konzentration von Analyten in Wasser bekannt. U. a. werden photometrische Testkits (Küvetten-Tests) und Teststreifen verwendet. Diesbezüglich sei beispielsweise auf die DE 199 06 151 A1, DE 196 760 A1, DE 43 078 14 A1, DE 100 18 784 C2, DE 10 12 199 A1, EP 0 663 239 B1 sowie WO 00/75653 verwiesen.

Küvettentests mit photometrischer Auswertung zeichnen sich dadurch aus, dass sie ein sehr verlässliches, digitales Ergebnis liefern, während die Vorteile der Teststreifen darin liegen, dass sie kostengünstig produziert werden können, einfach zu handhaben sowie zu entsorgen sind. Es ist erstrebenswert, die Vorteile beider Methoden in einem System zu verwirklichen. Demgemäß wird erfindungsgemäß eine Miniaturisierung des Küvettentests auf Teststreifengröße mit automatisierter Auswertung angestrebt.

Solche Systeme nutzen sogenannte "Indikatoren" zur Bestimmung der Analytkonzentration. Die Indikatoren erfahren aufgrund der Anwesenheit von Analytmolekülen eine optische Veränderung. Bei der optischen Veränderung des Indikators handelt es sich in der Regel um eine Veränderung der Lichtabsorption, der Fluoreszenz oder der Lumineszenz.

Gegenstand der Erfindung ist ein Verfahren zur Bestimmung der Konzentration von Analyten, wobei nacheinander

    i) der Analyt mit einem Indikator in Kontakt gebracht wird,
    ii) eine Bindung und/oder Anreicherung des Indikators, der mit dem Analyten reagiert hat, auf den magnetischen Partikeln stattfindet, sofern der Indikator nicht bereits auf den magnetischen Partikeln fixiert war,
    iii) nach der Reaktion des Indikators mit dem Analyten und der Bindung und/oder Anreicherung des Indikators auf den magnetischen Partikeln, sofern der Indikator nicht bereits auf den magnetischen Partikeln fixiert war, mittels eines Magnetfeldes eine Aufkonzentrierung der Partikel aus der Lösung erfolgt und
    iv) die Veränderung der magnetischen Partikel mittels optischer Methoden gemessen wird und wobei

    - der Indikator

        a) als Bestandteil eines Feststoffgemisches vorgelegt wird, das den Indikator und magnetische Partikel enthält und weitere Substanzen enthalten kann, oder
        b) als Bestandteil eines Feststoffgemisches vorgelegt wird, das den Indikator und magnetische Partikel enthält und weitere Substanzen enthalten kann, wobei der Indikator auf die Oberfläche der magnetischen Partikel aufgebracht ist, und

    - magnetische Partikel eingesetzt werden, welche einen Farbton aufweisen, welche nach dem CIELAB-System einen Wert von $L^* > 0$, insbesondere $L^* > 30$, aufweisen oder die weiß oder grau und/oder durchsichtig, bevorzugt weiß oder grau, sind.

Demgemäß sind die magnetischen Partikel also so ausgestaltet, daß sie eine optische, z.B. reflektometrische, Auswertung der Farbänderung zulassen.

Bei dem erfindungsgemäßen Verfahren werden Feststoffgemische (Reagenz) vorgelegt, welche magnetische Partikel und Indikatoren enthalten, aber auch noch weitere Substanzen enthalten können, wie zum Beispiel Puffersubstanzen, Maskierungsmittel oder Trägersubstanzen. Die Ummantelung der magnetischen Partikel ist vorteilhafterweise so gestaltet, dass sie genug hydrophile Bereiche hat, so dass sie im Wasser suspendierbar sind. Zusätzlich muss sie Bereiche haben, zu denen der Indikator eine höhere Affinität hat, als zu Suspensionsmedien, z B. zu Wasser. Dies kann z. B. durch kationische, anionische oder hydrophobe Wechselwirkungen oder durch molekulare Erkennung gewährleistet sein, bevorzugt durch kationische, anionische oder hydrophobe Wechselwirkungen.

Vorteilhaft ist es, wenn die Partikel superparamagnetisch sind, da sie dann nicht durch eigene Magnetisierung aneinander haften bleiben.

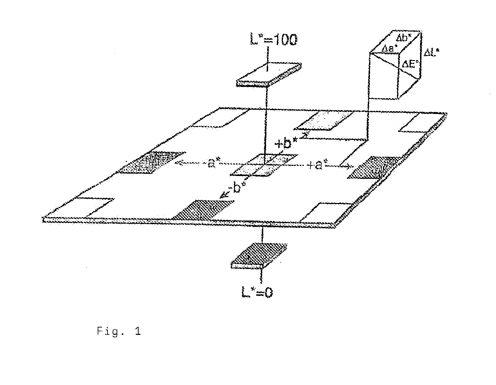

Erfindungsgemäß müssen die magnetischen Partikel derart ausgestaltet sein, dass mittels üblicher Messmethoden die Änderungen des auf ihnen angereicherten Indikators erkennbar sind. Insbesondere sollten Farbänderungen des Indikators mittels optischer Methoden bestimmbar sein. Zu diesem Zweck sollten magnetische Partikel eingesetzt werden, die eine möglichst helle Farbschattierung aufweisen. Durch den Einsatz vorzugsweise weißer, grauer, hell gefärbter und/oder durchsichtiger Partikel wird die Verwendung von aus dem Stand der Technik bekannten Farbindikatoren ermöglicht. Besonders bevorzugt sind weiße oder graue und/oder durchsichtige magnetische Partikel. Geeignet sind jedoch auch die verschiedenen Grauabstufungen zwischen schwarz und weiß. Neben den zwischen grau und schwarz liegenden Grauabstufungen sind auch vorzugsweise helle Farben geeignet, z. B. gelb oder auch grün. Die farbliche, inklusive Helligkeit, Eignung der magnetischen Partikel, d.h. solche, die im Rahmen der vorliegenden Erfindung zum Einsatz kommen, lässt sich beispielsweise anhand der CIELab-Farbabstandsformel bestimmen (vgl. RÖMPP - Lacke und Druckfarben 1998, Seite 114, vgl. auch Erläuterungen zur Fig 1). Erfindungsgemäß sind Partikel, welche schwarz sind, in der Regel kaum geeignet.

Unter grauen Partikeln werden im Rahmen der vorliegenden Erfindung solche Partikel verstanden, die die einen L*-Wert gemäß CIELab von >50, mehr bevorzugt >70, besonders bevorzugt >80 und insbesondere bevorzugt >90 aufweisen.

Erfindungsgemäß sind insbesondere auch graue magnetische Partikel einsetzbar, die auch einen Farbstich aufweisen. Trivialbezeichnungen für entsprechende Grautöne sind z.B. Aschgrau, Betongrau, Mausgrau, Rauchgrau, Sandgrau, Schiefergrau, Silbergrau, Zementgrau. Gut geeignet sind auch helle Brauntöne, wie z.B. beige o.ä.

In einer bevorzugten Ausgestaltung der vorliegenden Erfindung werden magnetische Partikel eingesetzt, die einen L*-Wert gemäß CIELab von >50, mehr bevorzugt >70, besonders bevorzugt >80 und insbesondere bevorzugt >90 aufweisen und dabei +a*, -a* und +b*, -b*-Werte gemäß CIELab aufweisen, deren Betrag jeweils unabhängig voneinander <70, bevorzugt <50, besonders bevorzugt <30, insbesondere bevorzugt <20, höchst bevorzugt <10 ist.

Die durchschnittliche Größe der magnetischen Partikel liegt zwischen 0,1 und 100$\mu$m, bevorzugt 0,2 bis 50$\mu$m, besonders bevorzugt 0,2 bis 20$\mu$m und insbesondere bevorzugt zwischen 0,7$\mu$m und 5$\mu$m. Das spezifische Gewicht der erfindungsgemäß bevorzugt einsetzbaren magnetischen Partikel liegt zwischen 1 und 10 g/m$^3$, insbesondere zwischen 2 und 7 g/m$^3$. Vorzugsweise sind die Partikel so gestaltet, dass sie in Wasser nur langsam sedimentieren und beim Durchmischen in Suspension gehalten werden können. Gleichzeitig sind die Partikel schwer genug, um innerhalb kurzer Zeit magnetisch aufkonzentriert (separiert) werden zu können. Vorzugsweise liegt das Reagenz mit den magnetischen Partikeln in trockener Form, z. B. als Lyophilisat vor.

Die magnetischen Partikel lassen sich ohne hohen Energie- oder mechanischen Aufwand aufkonzentrieren. Zur Aufkonzentrierung der magnetischen Partikel wird erfindungsgemäß nach der Reaktion des Indikators mit dem Analyten und der Bindung und/oder Anreicherung des Indikators auf den magnetischen Partikeln ein Magnetfeld eingesetzt. Die Magnetstärke des jeweils genutzten Magneten ist dabei an die jeweils eingesetzten magnetischen Partikel angepaßt. Die Flussdichte liegt bevorzugt zwischen 10 und 500mT, insbesondere zwischen 35 und 45mT.

Die magnetischen Partikel können Poren aufweisen, welche die spezifische Oberfläche erhöhen, in einer bevorzugten Ausführungsform haben die magnetischen Partikel Poren.

Die Poren sind von der Größe her so beschaffen, daß Analyten oder Analyt/Indikator-Komplex in sie hineindiffundieren können, so daß eine vergrößerte Oberfläche für die Anreicherung der Analyten oder Analyt/Indikator-Komplex zur Verfügung steht. Für die meisten herkömmlichen Indikatoren ist eine Porengröße von 60-100 Angström gut geeignet. Demgemäß ist es bevorzugt, wenn die Poren eine Größe von 40 bis 150, insbesondere 60-100, Angström aufweisen.

Die magnetischen Partikel gemäß vorliegender Erfindung weisen bevorzugt keine Lufteinschlüße auf.

Beispiele für erfindungsgemäß einsetzbare Magneten sind starke Permanentmagneten, wie Neodymium-Eisen-Bor-Magneten oder Samarium-Cobalt-Magneten. Ebenfalls einsetzbar sind Aluminium-Nickel-Cobalt-Magneten, Ferrit-Magneten (SrFe), Bismanol-Magneten, magnetischer Stahl oder kunststoffgebundenes magnetisches Material.

Es können im Rahmen der vorliegenden Erfindung auch Elektromagneten eingesetzt werden.

Bevorzugt werden im Rahmen der vorliegenden Erfindung Permanentmagneten eingesetzt.

Als Indikatoren können die aus dem Stand der Technik bekannten Stoffe verwendet werden. Besonders bevorzugt sind Indikatoren, die in der Photometrie verwendet werden (s. z.B. Z. Marczenko, Separation and Spectrophotometric Determination of Elements, 1986, p. 1-678:

- N,N- Diethyl-1,4-Benzenediamine (DPD) und Tetramethylbenzidine zur quantitativen Bestimmung von Chlor, Brom, Iod, Ozon, Chlordioxid in Wasser,
- Bathocuproindisulfonsäure-Dinatriumsalz für Kupfer,
- Phenantrolin für Eisen,
- Metallphthalein für Härte,
- Zincon für Kupfer,
- Pyridyldiphenyltriazin für Eisen usw.).

Die Farbänderung der Indikatoren kann mit üblichen Methoden gemessen werden. Erfindungsgemäß handelt es sich hierbei um optische Methoden. Beispiele hierfür sind Photometrie, Reflektometrie, Flourimetrie, Luminometrie und Colorimetrie. Insbesondere bevorzugt im Rahmen der vorliegenden Erfindung ist die Reflektometrie.

Gegenstand der Erfindung ist schließlich auch die Verwendung von geeigneten magnetischen Partikeln für die Bestimmung der Konzentration von Analyten, insbesondere bei der Analyse von Wasserproben. Diese Partikel sind in der zuvor beschriebenen Form ausgestaltet.

Die vorliegende Erfindung betrifft insbesondere auch ein Verfahren zur Konzentrationsbestimmung von Analyten, bei dem die Analyten chemische Parameter in Wässern sind.

Die Wässer sind dabei bevorzugt ausgewählt aus der Gruppe bestehend aus Trinkwasser, Abwasser, Prozeßwasser, Kesselwasser, Kühlwasser, Schwimmbadwasser, Badewasser und Gewässern.

Als Gewässer sind zu verstehen: Meere, Seen, Teiche, Tümpel, Moore, Flüsse, Bäche, Quellen, Aquarien beliebiger Größe, insbesondere Seen, Teiche, Flüsse, Bäche und Aquarien.

Chemische Parameter sind dabei zum Beispiel:

- Kationen, insbesondere Ammonium, Magnesium, Calcium, Eisen, Kupfer,
- Anionen, insbesondere Nitrat und Phosphat
- neutrale Analyten, insbesondere Chlor, Brom, Iod, Ozon, Chlordioxid und Gemische davon.

In einer Variante der vorliegenden Erfindung kann der chemische Parameter auch der pH-Wert (entsprechend $H^+/H_3O^+$-Kationen) sein.
Ferner kann der chemische Parameter Alkalinität und/oder Wasserhärte sein.
In einer Variante der vorliegenden Erfindung kann der Analyt ein Enzym sein und der Indikator ein Substrat für das Enzym.
Dabei sind Galactosidase oder Glucuronidase Beispiele für Enzyme als Analyten.
Entsprechende Indikatoren wären für diese beiden zum Beispiel Methylumbelliferyl Galactose für die Galactosidase und 4-Methylumbelliferyl beta-D-Glucuronid für die Glucuronidase.
Auf diese Art und Weise ist mit dem erfindungsgemäßen Verfahren eine Quantifizierung von Enzymen möglich.

Demgemäß umfaßt diese Variante der vorliegenden Erfindung auch ein Verfahren zur Quantifizierung von Enzymen, bei dem der Analyt ein Enzym, der Indikator ein Substrat für das Enzym und der Indikator, der mit dem Analyten reagiert hat, das Produkt der Enzymreaktion ist.
Dadurch ist es mittels dieser Variante des erfindungsgemäßen Verfahrens ferner möglich, Mikroorganismen nachzuweisen und zu quantifizieren, indem die von den Mikroorganismen produzierten Enzyme bestimmt werden.
Diese Variante der vorliegenden Erfindung eignet sich insbesondere auch für die Quantifizierung von Enzymen und/oder Mikroorganismen in Wasserproben aus Trinkwasser, Abwasser, Schwimmbadwasser, Badewasser und Gewässern.

Es ist demgemäß im Rahmen der vorliegenden Erfindung auch möglich durch geeignete Wahl der eingesetzten Indikatoren, insbesondere durch Indikatoren, die verschiedene Absorptions- und/oder Transmissionsspektren beziehungsweise Reflektionsspektren aufweisen, mehrere, bevorzugt zwei oder drei, insbesondere zwei, Analyten, nebeneinander zu bestimmen.

Das vorliegende Verfahren wird bevorzugt mittels einem Einfach-Analysengerät ausgeführt, d.h. als Analysengerät, welches einzeln eingesetzt wird und nicht im apparative Verbund mit anderen.
Dieses Analysengerät kann als wiederverwertbares Analysengerät ausgestaltet sein oder als Einmal-Analysengerät, d.h. ein Analysengerät, das nach einmaligem Gebrauch entsorgt wird, es ist bevorzugt, daß das Analysengerät als Einmal-Analysengerät ausgestaltet ist.

Bevorzugt sind die Analysengeräte im Rahmen der vorliegenden Erfindung derart ausgestaltet, daß sie

- eine Öffnung, durch die die Probe aufgenommen wird,
- einen Fleck, an dem das Reagenz bereitgestellt wird (1),
- einen Kanal, in dem Probe und Reagenz durchmischt wird (2), und
- einen Meßfleck (3), an dem die magnetischen Partikel gesammelt werden,
- eine Pumpkammer (4), oder
- statt einer Pumpkammer eine externe Pumpvorrichtung oder einen Anschluß für eine Pumpvorrichtung,

umfassen.

Der Fleck, an dem das Reagenz bereitgestellt wird, sowie der Meßfleck können dabei entweder an den Enden des Kanals angeordnet sein, oder aber, bevorzugt in dem Kanal liegen.
In einer Ausführungsform ist die Öffnung zur Probenaufnahme der Küvette so gestaltet, daß sie erst unmittelbar vor der Probennahme geöffnet wird.

In einer bevorzugten Ausgestaltung kann die Pumpkammer in Form einer Pumpblase, die z.B. mittels eines Gegenstandes oder eines Fingers betätigt werden kann, mit deren Hilfe die Probe also mittels Drücken und Loslassen, durch den Kanal gepumpt werden kann, ausgestaltet sein.

Die Einmal-Analysengeräte können im Rahmen der vorliegenden Erfindung auch die Größendimensionen handelsüblicher Teststreifen aufweisen; jedoch ist es ebenso möglich, daß sie deutlich größer sind, z.B. die ungefähre Größe einer Zigarettenschachtel, eines Feuerzeugs, einer Scheckkarte aufweisen.
In einer bevorzugten Variante weisen die Analysengeräte dabei eine Breite zwischen 3mm und 50mm, bevorzugt zwischen 5 und 20mm und eine Höhe von 20mm bis 60mm, bevorzugt 25 bis 50mm.
Die Analysengeräte können dabei eine Dicke von 0,5 bis 20mm, bevorzugt 0,5 bis 10mm, besonders bevorzugt 0,5 bis 7mm, insbesondere bevorzugt 0,7 bis 5mm und höchst bevorzugt 1 bis 3mm aufweisen.

Eine erfindungsgemäß einsetzbares Analysengerät weist beispielsweise eine Breite zwischen 5 und 15mm, eine Höhe zwischen 25 und 45mm und eine Dicke von 1 bis 10mm auf.

Die optische Auswertung erfolgt im Rahmen der vorliegenden Erfindung während/wenn die magnetischen Partikel mit den daran gebundenen Analyten magnetisch gesammelt sind, d.h. sie liegen bei der Auswertung nicht gelöst oder dispergiert vor.
Ein gesonderter Schritt zum Waschen bzw. Reinigen der Partikel ist im Rahmen der vorliegenden Erfindung nicht notwendig. Bevorzugt erfolgt die vorliegende Erfindung ohne einen solchen Schritt.

Als Lichtquelle für die optische Auswertung kommen im Rahmen der vorliegenden Erfindung bevorzugt solche ausgewählt aus der Gruppe bestehend aus Lasern, Lampen, LED's, OLED's in Frage.

Es ist bei der Messung im Rahmen der vorliegenden Erfindung unerheblich, ob auf den magnetischen Partikeln neben den an den Indikator gebundenen Analytmolekülen/-ionen noch Indikator adsobiert bzw. fixiert ist, der nicht mit dem Analyten reagiert hat, oder nicht; beide Varianten sind von der vorliegenden Erfindung umfaßt. Anders ausgedrückt: unbenutzter Indikator stört die Messung nicht und kann demgemäß unberücksichtigt bleiben.

Dies ist auch notwendig, da vor der erfindungsgemäßen Quantifizierung des Analyten nicht bekannt ist, welche Menge Indikator nötig wäre.

Gegenstand der vorliegenden Erfindung sind auch die vorstehend beschriebenen magnetischen Partikel, sowie deren Verwendung zur Bestimmung der Konzentration von Analyten, vorzugsweise in Wasser.

Im Folgenden wird die Erfindung unter Bezugnahme auf die Figuren beispielhaft näher erläutert:

Fig. 1 zeigt die Konstruktion des Farbabstands im CIELAB-System

Fig. 2 zeigt die Aufsicht auf einen Chip, der zur Konzentrationsmessung von Analyten verwendet werden kann.

Das System zur Fig. 1 bietet ein Verfahren zur quantitativen Bestimmung von Farbabständen bei Körperfarben. Der Farbabstand $\Delta E_{ab}$ nach DIN 6174: 1979 - 01 im CIELAB-System wird als Raumdiagonale berechnet. Die an der genannten Literaturstelle angegebene Formel lautet:

$$\Delta E_{ab} = [(\Delta L^*)^2 + (\Delta a^*)^2 + (\Delta b^*)^2]^{1/2}.$$

Der Farbenraum wird durch L\*, a\*, b\* bestimmt. L\*=0 bedeutet schwarz. L\*=100 ist weiß. Dazwischen liegen die verschiedenen Grauabstufungen. -b\* sind die Abstufen zu blau, +b\* zu gelb, -a\* zu grün und +a\* zu rot. Erfindungsgemäß kommen daher insbesondere die Bereiche L\*>0 bis L\*=100 sowie +b\* und -a\* in Betracht. Höchst bevorzugt sind L\*>70, L\*=100 sowie durchsichtige Varianten.

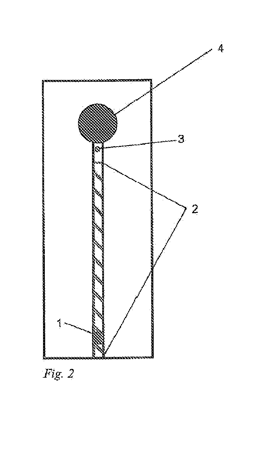

Zur Quantifizierung der Konzentration eines Analyten werden 10 μl einer Probe in eine mikrofluidische Kanalstruktur aus Kunststoff eingesaugt. Diese ist schematisch in Fig. 2 dargestellt. Es handelt sich um die Aufsicht auf einen Chip, der zur Konzentrationsbestimmung von Analyten verwendet werden kann. Das Reagenz, welches magnetische Partikel und Indikator enthält wird in fester Form an Position 1 vorgelegt. In dem Bereich 2 erfolgt die Durchmischung mit der Probe. Mittels des Magneten 3 erfolgt die Aufkonzentrierung der Partikel auf einen Messfleck. Die Kanalstruktur besteht aus einem 20 mm langen und 1 x 1 mm² großen Kanal, der an einem Anschluss für eine Pumpe endet. Durch Betätigen einer Pumpe 4 (gegebenenfalls mehrmals) wird die Probe in und durch den Kanal bewegt.

Es ist im Rahmen der vorliegenden Erfindung möglich, als Pumpe 4 eine beliebige Pumpenform/-art einzusetzen, eine Pumpblase, eine Hubkolbenpumpe oder eine peristaltische Pumpe, insbesondere eine Pumpblase.

Ein Reagenz mit Puffersalzen und 0,064 μl weißen, magnetischen Partikeln 1 liegt in fester Form oder als Lyophilisat im Bereich nahe der Kanalöffnung vor und wird durch Hin- und Herpumpen der Probenflüssigkeit innerhalb der 18 μl Kanalstruktur, die dem Eingang des Kanals zugewandt sind, gelöst bzw. suspendiert und durchmischt. Die Partikel haben in diesem Beispiel einen Durchmesser von ca. 1 μm.

Die Volumina der eingesetzten Probe und der Menge an magnetischen Partikeln, sowie die genauen Dimensionen des fluidischen Kunststoffchips mit der Pumpe kann aber im Rahmen der vorliegenden Erfindung an unterschiedliche Aufgabenstellungen angepaßt werden.

Während der Durchmischung, die z.B. während 30s erfolgt, reagiert der Indikator mit dem Analyten zu einer Substanz, die anders gefärbt ist, oder anders reflektiert als die Ausgangsmaterialien (ein Analyt-Indikator.Komplex wird gebildet).

Gleichzeitig wird das Reaktionsprodukt aus Indikator und Analyt an den weißen magnetischen Partikeln adsorbiert, sofern der Indikator nicht bereits vorher auf der Oberfläche des magnetischen Partikels fixiert war.

Anschließend wird die Suspension weiter in Richtung der Pumpe, bzw. bis in die Pumpe hinein, gezogen und dabei über ein 0,4 x 0,4 mm² großes Magnetfeld bewegt, das zu diesem Zeitpunkt an der Unterseite des Chips außerhalb des Kanals angelegt ist. Dabei werden die magnetischen Partikel an dem Magnetfeld auf einem Messfleck von 0,4 x 0,4 mm² abgeschieden.

Das Magnetfeld wird dabei an dem Ort des Meßflecks durch einen Permanentmagneten erzeugt, kann aber auch durch einen Elektromagneten erzeugt werden. Alternativ kann auch ein Magnet an der Unterseite der Kanalstruktur vorbei bewegt werden, wodurch die Partikel eingesammelt werden.

Dabei werden die magnetischen Partikel an dem Magnetfeld auf einem Meßvolumen von 0,4 x 0,4 x 0,4 mm³ (= 0,064 μl) abgeschieden.

Durch die Aufkonzentrierung des Indikators von einem Probevolumen von 10 μl auf ein Partikelvolumen von 0,064 μl sind diese bei einem Farbindikator deutlich intensiver gefärbt als die Lösung.

Die Farbintensität wird dann colorimetrisch in Reflexion gemessen. Da diese mit der Konzentration des Analyten korelliert, kann von ihr auf die Konzentration des Analyten geschlossen werden.

**Patentansprüche**

1.  Verfahren zur Konzentrationsbestimmung von Analyten, wobei nacheinander

    i) der Analyt mit einem Indikator in Kontakt gebracht wird,
    ii) eine Bindung und/oder Anreicherung des Indikators, der mit dem Analyten reagiert hat, auf den magnetischen Partikeln stattfindet, sofern der Indikator nicht bereits auf den magnetischen Partikeln fixiert war,
    iii) nach der Reaktion des Indikators mit dem Analyten und der Bindung und/oder Anreicherung des Indikators auf den magnetischen Partikeln, sofern der Indikator nicht bereits auf den magnetischen Partikeln fixiert war, mittels eines Magnetfeldes eine Aufkonzentrierung der Partikel aus der Lösung erfolgt und
    iv) die Veränderung der magnetischen Partikel mittels optischer Methoden gemessen wird und wobei

    - der Indikator

    a) als Bestandteil eines Feststoffgemisches vorgelegt wird, das den Indikator und magnetische Partikel enthält und weitere Substanzen enthalten kann, oder
    b) als Bestandteil eines Feststoffgemisches vorgelegt wird, das den Indikator und magnetische Partikel enthält und weitere Substanzen enthalten kann, wobei der Indikator auf die Oberfläche der magnetischen Partikel aufgebracht ist, und

    - magnetische Partikel eingesetzt werden, welche einen Farbton aufweisen, welche nach dem CIELAB-System einen Wert von L*>30 aufweisen oder die weiß oder grau und/oder durchsichtig, bevorzugt weiß oder grau, sind.

2.  Verfahren nach Anspruch 1 **dadurch gekennzeichnet, dass** der Indikator auf die Oberfläche der magnetischen Partikel aufgebracht ist, insbesondere in Form einer Beschichtung.

3.  Verfahren nach einem der vorhergehenden Ansprüche **dadurch gekennzeichnet, dass** Partikel eingesetzt werden, deren Oberfläche Bereiche aufweist, zu denen der Indikator eine höhere Affinität als zum Suspensionsmedium hat.

4.  Verfahren nach einem der vorhergehenden Ansprüche **dadurch gekennzeichnet, dass** Indikatoren verwendet werden, deren Farbänderung messbar ist.

5.  Verfahren nach einem der vorhergehenden Ansprüche **dadurch gekennzeichnet, dass** die magnetischen Partikel eine Größe von 0,1 bis 100 $\mu$m haben.

6.  Verfahren nach einem der vorhergehenden Ansprüche **dadurch gekennzeichnet, dass** eine magnetische Flussdichte von 10 bis 500mT zur Aufkonzentrierung mittels magnetischer Filtration verwendet wird.

7.  Verfahren nach einem der vorhergehenden Ansprüche **dadurch gekennzeichnet, dass** die Indikatoränderung photometrisch, colorimetrisch, luminometrisch, flourimetrisch oder reflektrometrisch, insbesondere reflektrometrisch, gemessen wird.

8.  Magnetische, suspendierbare Partikel zur Durchführung des Verfahrens nach einem der vorhergehenden Ansprüche, wobei die Partikel nach der CIELAB-Farbabstandsformel einen Wert von L*>30 aufweisen oder die weiß oder grau und/oder durchsichtig, bevorzugt weiß oder grau, sind und deren Oberfläche eine höhere Affinität zum Indikator als zum Suspensionsmedium aufweist und wobei sie eine Größe von 0,1 bis 100 $\mu$m aufweisen.

9.  Partikel nach Anspruch 8 **dadurch gekennzeichnet, dass** sie mit einem Indikator beschichtet sind oder als Gemisch mit einem Indikator vorliegen.

10. Partikel nach Anspruch 8 **dadurch gekennzeichnet, dass** deren Oberfläche hydrophile Bereiche aufweist.

11. Partikel nach einem der vorhergehenden Ansprüche 8 bis 10 **dadurch gekennzeichnet, dass** sie in trockener, fester Form vorliegen.

12. Partikel nach einem der vorhergehenden Ansprüche 9 bis 11 **dadurch gekennzeichnet, dass** Indikator und magnetische Partikel in Form eines Lyophilisats vorliegen.

13. Verwendung der Partikel nach einem der Ansprüche 8 bis 12 zur Bestimmung der Konzentration von Analyten, vorzugsweise in Wasser.

Fig. 1

Fig. 2

**Europäisches Patentamt**

**EUROPÄISCHER RECHERCHENBERICHT**

Nummer der Anmeldung

EP 08 10 3540

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2003/077598 A1 (PHAN BRIGITTE CHAU [US] ET AL) 24. April 2003 (2003-04-24) <br> * Zusammenfassung * <br> * Absätze [0006], [0025], [0027], [0142], [0147] - [0154], [0186] * <br> * Absätze [0010], [0045]; Abbildung 20C * | 1-11 | INV. <br> G01N33/18 <br> G01N35/00 <br><br> ADD. <br> G01N31/22 |
| X | * Absatz [0148] * | 12 | |
| X | * Absatz [0015] * | 13 | |
| | ----- | | |
| X | EP 0 893 692 A1 (STRATEC ELEKTRONIK GMBH [DE]; STRATEC BIOMEDICAL SYSTEMS AG [DE]) 27. Januar 1999 (1999-01-27) <br> * Zusammenfassung * <br> * Spalte 1, Zeilen 12-32 * <br> * Spalte 4, Zeile 53 - Spalte 6, Zeile 1 * | 1,8,13 | |
| | ----- | | |
| X | US 2005/191687 A1 (WANG TIANXIN [US] ET AL) 1. September 2005 (2005-09-01) <br> * Zusammenfassung; Abbildung 6 * <br> * Absätze [0006], [0063] * | 1,8,13 | |
| X | * Absatz [0040] * | 10 | RECHERCHIERTE SACHGEBIETE (IPC) |
| | ----- | | |
| X | DE 10 2004 044048 A1 (QUALIS LAB GMBH [DE]) 30. März 2006 (2006-03-30) <br> * Zusammenfassung; Abbildungen A,B,C * <br> * Absatz [0002]; Anspruch 1 * | 1,13 | G01N |
| | ----- | | |
| X | US 4 731 337 A (LUOTOLA JUHANI E I [FI] ET AL) 15. März 1988 (1988-03-15) <br> * Zusammenfassung * <br> * Spalte 1, Zeilen 5-9,31-34 * | 8 | |
| | ----- | | |
| A | US 6 254 830 B1 (PIVARNIK PHILIP [US] ET AL) 3. Juli 2001 (2001-07-03) <br> * Zusammenfassung * <br> * Spalte 2, Zeilen 54-64 * <br> * Spalte 6, Zeilen 31-34,45-47 * | 1-13 | |
| | ----- | | |

Der vorliegende Recherchenbericht wurde für alle Patentansprüche erstellt

| Recherchenort | Abschlußdatum der Recherche | Prüfer |
|---|---|---|
| München | 31. Juli 2008 | Hanisch, Christian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

..............................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**                    EP 08 10 3540

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-07-2008

| Im Recherchenbericht angeführtes Patentdokument | Datum der Veröffentlichung | Mitglied(er) der Patentfamilie | Datum der Veröffentlichung |
|---|---|---|---|
| US 2003077598 A1 | 24-04-2003 | US 2005266476 A1 | 01-12-2005 |
| EP 0893692 A1 | 27-01-1999 | KEINE | |
| US 2005191687 A1 | 01-09-2005 | WO 2005085850 A1 | 15-09-2005 |
| DE 102004044048 A1 | 30-03-2006 | KEINE | |
| US 4731337 A | 15-03-1988 | DE 3584196 D1<br>EP 0169434 A2<br>JP 2584611 B2<br>JP 61041966 A | 31-10-1991<br>29-01-1986<br>26-02-1997<br>28-02-1986 |
| US 6254830 B1 | 03-07-2001 | US 6630355 B1 | 07-10-2003 |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 07106535 A
- DE 19906151 A1
- DE 196760 A1
- DE 4307814 A1
- DE 10018784 C2
- DE 1012199 A1
- EP 0663239 B1
- WO 0075653 A

**In der Beschreibung aufgeführte Nicht-Patentliteratur**

- *RÖMPP - Lacke und Druckfarben,* 1998, 114
- **Z. MARCZENKO.** *Separation and Spectrophotometric Determination of Elements,* 1986, 1-678